(19) Europäisches Patentamt
European Patent Office
Office européen des brevets

(11) EP 1 077 059 A2

(12) EUROPÄISCHE PATENTANMELDUNG

(43) Veröffentlichungstag:
**21.02.2001 Patentblatt 2001/08**

(51) Int. Cl.[7]: **A61K 7/00**

(21) Anmeldenummer: **00116483.9**

(22) Anmeldetag: **29.07.2000**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **16.08.1999 DE 19938757**

(71) Anmelder: **Beiersdorf AG**
**20245 Hamburg (DE)**

(72) Erfinder:
- **Müller, Anja**
  **23843 Rümpel (DE)**
- **Gers-Barlag, Heinrich, Dr.**
  **25495 Kummerfeld (DE)**

(54) **Kosmetische oder dermatologische Zubereitungen von Typ Öl-in-Wasser**

(57) Dünnflüssige kosmetische oder dermatologische Zubereitungen vom Typ Öl-in-Wasser, welche eine Ölphase, in welche hydrophobe und/oder amphiphile Feststoffe eingearbeitet sind, und eine Wasserphase enthalten, wobei die Dichtedifferenz zwischen der Ölphase und der Wasserphase (bestimmbar mit einem rechnenden digitalen Dichtemesser vom Typ DMA 45 der Firma chempro/PA bei 25 °C) nicht größer als 0,01 $g \cdot cm^{-3}$ ist und Verfahren zur Stabilisierung von O/W-Formulierungen.

EP 1 077 059 A2

Printed by Xerox (UK) Business Services
2.16.7 (HRS)/3.6

## Beschreibung

Die vorliegende Erfindung betrifft kosmetische und dermatologische Zubereitungen vom Typ Öl-in-Wasser, insbesondere sprühbare O/W-Emulsionen, welche eine Viskosität von weniger als 2000 mPa • s haben, sowie ein Verfahren zur Stabilisierung von O/W-Formulierungen mittels Dichteangleich der Phasen.

Kosmetische Zubereitungen werden im wesentlichen zur Pflege der Haut benutzt. Die menschliche Haut übt als größtes Organ des Menschen zahlreiche lebenswichtige Funktionen aus. Mit durchschnittlich etwa 2 $m^2$ Oberfläche beim Erwachsenen kommt ihr eine herausragende Rolle als Schutz- und Sinnesorgan zu. Unter ihren vielen Funktionen (beispielsweise zur Wärmeregulation) ist die Barrierefunktion, die das Austrocknen der Haut (und damit letztlich des gesamten Organismus) verhindert, die wohl wichtigste. Gleichzeitig wirkt die Haut als Schutzeinrichtung gegen das Eindringen und die Aufnahme von außen kommender Stoffe (z. B. Schmutz, Chemikalien, Mikroorganismen). Außerdem kommt ihr eine bedeutende Rolle als Regulations- und Zielorgan im menschlichen Stoffwechsel zu.

Die kosmetische Hautpflege dient in erster Linie dazu, die natürliche Funktion der Haut als Barriere gegen Umwelteinflüsse und gegen den Verlust von körpereigenen Stoffen (neben Wasser auch natürliche Fette, Elektrolyte etc.) zu stärken oder wiederherzustellen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die schädigende Wirkung des ultravioletten Teils der Sonnenstrahlung auf die Haut ist allgemein bekannt. In Abhängigkeit von ihrer jeweiligen Wellenlänge haben die Strahlen verschiedene Wirkungen auf das Organ Haut: Die sogenannte UV-C-Strahlung mit einer Wellenlänge, die kleiner als 290 nm ist, wird von der Ozonschicht in der Erdatmosphäre absorbiert und hat keine physiologische Bedeutung. Dagegen verursachen Strahlen im Bereich zwischen 290 nm und 320 nm, dem sogenannten UV-B-Bereich, ein Erythem, einen einfachen Sonnenbrand oder sogar mehr oder weniger starke Verbrennungen. UV-A-Strahlung (320 bis 400 nm) ist im Hinblick auf die Auslösung photodynamischer, speziell phototoxischer Reaktionen und chronischer Veränderungen der Haut noch weitaus gefährlicher als UV-B-Strahlung. So reicht selbst die UV-A-Strahlung unter ganz normalen Alltagsbedingungen aus, um innerhalb kurzer Zeit die Kollagen- und Elastinfasern zu schädigen. Auch kann der schädigende Einfluß der UV-B-Strahlung durch UV-A-Strahlung noch verstärkt werden.

Ferner können bereits sehr geringe Strahlendosen photochemische Reaktionen auslösen. Hierzu gehört insbesondere die Bildung freier Radikale, welche wiederum aufgrund ihrer hohen Reaktivität unkontrollierte Folgereaktionen auslösen können. Um solchen Reaktionen vorzubeugen, können kosmetischen bzw. dermatologischen Formulierungen neben UV-Filtersubstanzen zusätzlich Antioxidantien und/oder Radikalfänger zugesetzt werden.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z. B. Kosmetikverordnung, Lebensmittel- und Arzneimittelgesetz).

Den bei weitem wichtigsten Produkttyp im Bereich der Hautpflegemittel stellen Emulsionen dar. Emulsionen sind disperse Zwei- oder Mehrphasensysteme, wobei kosmetische Emulsionen aus mindestens einer Fettphase (Fette und mineralische Öle, Fettsäureester, Fettalkohole etc.) und mindestens einer Wasserphase (Wasser, Glycerin, Glykole usw.) bestehen, die mit Hilfe von Emulgatoren in Form feinster Tröpfchen ineinander verteilt werden. Liegt die Ölphase fein verteilt in der Wasserphase vor, so handelt es sich um eine Öl-in-Wasser-Emulsion (O/W-Emulsion, z. B. Milch). Der Grundcharakter einer O/W-Emulsion ist durch das Wasser geprägt, d. h. sie wirkt weniger fettend auf der Haut, ist eher mattierend und zieht schneller in die Haut ein als eine W/O-Emulsion.

Obwohl Emulsionen vom thermodynamischen Standpunkt aus betrachtet instabile Systeme sind, gelingt es, kosmetische Emulsionen von jahrelanger Stabilität herzustellen. Eine Ermitsion wird als stabil bezeichnet, wenn sich über einen vorgegebenen Zeitraum hinweg keine meßbaren zeitlichen und örtlichen Änderungen der Tropfengräßenverteilung feststellen lassen.

Die Stabilität bzw. Instabilität von Emulsionen hängt von verschiedenen Faktoren ab. Zum einen neigt beispielsweise die Wasserphase einer W/O-Emulsion, da Wasser- und Ölphase unterschiedliche Dichten haben, zur Sedimentation. Die Ölphase einer O/W-Emulsion hat dementsprechend eine Tendenz zum Aufrahmen.

Ferner kann es aufgrund der Anziehungskräfte zwischen, den feinverteilten Tröpfchen der dispersen Phase zu einer Tropfenaggregation kommen, wobei die einzelnen Tröpfchen eines Aggregates zunächst durch einen dünnen Film kontinuierlicher Phase voneinander getrennt bleiben. Die ursprüngliche Tropfengrößenverteilung ändert sich dabei nur scheinbar und kann in diesem Fall durch Rühren oder Schütteln wiederhergestellt werden.

Allerdings können die sich berührenden Tröpfchen darüber hinaus auch zusammenfließen, was zu einer echten Änderung der Tropfengrößenverteilung führt, die nur durch Energiezufuhr wieder verändert werden kann. Dieser Vorgang wird als Koaleszenz bezeichnet. Der Prozeß der Koaleszenz läuft um so langsamer ab, je viskoser die

äußere Phase der Emulsion ist.

Die beschriebenen Vorgänge können einzeln oder zusammen ablaufen. Oft initiiert oder verstärkt ein Vorgang den anderen. So wird z. B. durch die Bildung von Aggregaten in O/W-Emulsionen das Aufrahmen der Ölphase beschleunigt. Geht der disperse Zustand einer Emulsion teilweise oder auch ganz verloren, so trennen sich die beiden Phasen, und man spricht vom Brechen der Emulsion.

Zur Stabilisierung von Emulsionen über einen längeren Zeitraum hinweg werden dementsprechend Hilfsmittel benötigt, die das Entmischen der beiden Phasen unterbinden, mindestens aber so lange verzögern, bis die Emulsion ihre Bestimmung erfüllt hat.

Diese Hilfsmittel sollen zum einen die Grenzfläche stabilisieren, indem sie verhindern, daß die Tröpfchen der dispersen Phase zusammenfließen. Im Idealfall bewirken diese Stoffe darüber hinaus eine Abstoßung der Tröpfchen, welche verhindert, daß sich diese annähern, so daß eine Zusammenballung (Aggregatbildung) vermieden werden kann.

Zum anderen werden Hilfsstoffe dazu eingesetzt, dem Aufrahmen bzw. Sedimentieren der Phasen entgegenzuwirken.

Emulgatoren sind grenzflächenaktive Substanzen, die in der Lage sind, die Grenzflächenspannung zwischen Öl- und Wasserphase zu vermindern, indem sie sich bevorzugt an der Grenzfläche zwischen diesen beiden anlagern. Dies wird durch ihren amphipilen Molekülaufbau ermöglicht: Emulgatoren besitzen wenigstens eine polare (hydrophile) Gruppe und wenigstens eine unpolare (lipophile) Gruppe. Damit sind sie sowohl in der hydrophilen als auch in der lipophilen Phase löslich. Der in der entsprechenden Phase besser lösliche Teil ragt in diese hinein und senkt dadurch die Grenzflächenspannung zwischen beiden Phasen.

Der Versuch der Klassifizierung von Emulgatoren ist schwierig, da diese zu chemisch sehr unterschiedlichen Kategorien gehören. Ein Emulgator ist um so effektiver, je schneller er die Grenzflächenspannung erniedrigt und je niedriger der Gleichgewichtswert der Grenzflächenspannung ist.

Darüber hinaus stabilisieren Emulgatoren auch durch Ausbildung von Grenzflächenfilmen und damit „physikalischen" Barrieren, wodurch die Aggregatbildung und die Koaleszenz der emulgierten Teilchen verhindert wird. Durch die Anlagerung des Emulgators an der Grenzfläche werden die Tröpfchen entweder aufgeladen, so daß sie sich gegenseitig abstoßen, oder es wird eine stabile, vielfach hochviskose oder sogar feste Schutzschicht um die Tröpfchen gebildet.

Für die praktische Herstellung kosmetischer oder dermatologischer Emulsionen reicht allerdings in der Regel der Einsatz eines oder mehrerer Emulgatoren allein nicht aus. Wesentliche Faktoren für die Stabilität kosmetischer oder dermatologischer Zubereitungen sind ferner:

- feinste Verteilung der beiden Phasen ineinander Je kleiner die dispergierten Teilchen sind, um so stabiler ist die Emulsion.
- hohe Viskosität der äußeren Phase
- ein stabiler Grenzflächenfilm
- ein ausgewogenes Phasenvolumenverhältnis

Das Emulgatorsystem muß daher meist zusätzlich zum eigentlichen Emulgator noch eine weitere Komponente enthalten, die als Co-Emulgator, Stabilisator oder je nach Wirkmechanismus auch als Konsistenzgeber, Verdickungsmittel oder Schutzkolloid usw. bezeichnet wird.

Durch diese Stoffe, im folgenden der Einfachheit halber Stabilisatoren genannt, wird die Stabilität einer Emulsion erhöht. Stabilisatoren müssen nicht grenzflächenaktiv, können aber amphiphil aufgebaute Verbindungen sein.

Eine Möglichkeit, Emulsionen zu stabilisieren, ist nach dem oben gesagten, die Viskosität der äußeren Phase zu erhöhen. Diese Viskositätserhöhung bewirkt in der Regel eine erhebliche Verminderung der Beweglichkeit der dispergierten Tröpfchen, wodurch die Sedimentations- bzw. Aufrahmgeschwindigkeit vermindert wird. Damit einhergehend treffen die Tröpfchen ferner weniger häufig aufeinander, was eine geringere Koaleszenzneigung zur Folge hat.

Die Viskosität der äußeren Phase läßt sich beispielsweise durch Zugabe von Verdickungsmitteln erhöhen, welche z. B. Gele und/oder lamellare Flüssigkristalle ausbilden. Auch Emulgatoren sind im Prinzip in der Lage, durch die Bildung von Emulgatorgelnetzwerken die Viskosität einer Flüssigkeit zu erhöhen. Allerdings wird hierfür eine vergleichsweise hohe Menge an Emulgator benötigt, da Gelnetzwerke erst dann gebildet werden, wenn die gesamte Grenzfläche zwischen den Phasen mit Emulgatormolekülen belegt ist.

Das Brechen einer Emulsion läßt sich ferner durch Wahl eines geeigneten Phasenvolumenverhältnisses verhindern. Zur Verdeutlichung dieser Tatsache stelle man sich eine Emulsion als System aus Metallkugeln gleichen Durchmessers (innere Phase) und einer Flüssigkeit (äußere Phase) vor. Eine Sedimentation bzw. ein Aufrahmen kann - in diesem einfachen Modell - dann nicht mehr stattfinden, wenn die gesamte Flüssigkeit mit Metallkugeln ausgefüllt

ist. Dies ist - nimmt man eine dichteste Kugelpackung als Verteilung an - gerade bei einem Verhältnis von 1 : 2 der Fall, d. h. wenn die Emulsion zu 2/3 aus innerer Phase besteht. Es ist offensichtlich, daß die Viskosität einer Emulsion bei wachsendem Anteil an innerer Phase zunimmt, da hierdurch die Beweglichkeit der dispergierten Tröpfchen eingeschränkt wird.

Natürlich ist dem Fachmann eine Vielzahl von Möglichkeiten bekannt, stabile O/W-Zubereitungen zur kosmetischen oder dermatologischen Anwendung zu formulieren, beispielsweise in Form von Cremes und Salben, die im Bereich von Raum- bis Hauttemperatur streichfähig sind, oder als Lotionen und Milche, die in diesem Temperaturbereich eher fließfähig sind. Dabei kommt es neben der Wahl des „richtigen" Emulgators bzw. Emulgatorsystems insbesondere auch auf die weitere Zusammensetzung der Zubereitung an.

O/W-Emulsionen werden in der Regel durch Verdickungsmittel, welche die Viskosität der wäßrigen Phase erhöhen, stabilisiert. Hierzu eignen sich beispielsweise Polyacrylate (Carbomer) und weitere organische Verdickungsmittel. Ein Nachteil dieser Methode der Stabilitätsverbesserung ist die Empfindlichkeit dieser Formulierungen gegen Elektrolyte. Ferner sind auf diese Weise naturgemäß vornehmlich höherviskose Formulierungen (wie Cremes oder Salben) herzustellen. Auch die Stabilisierung von O/W-Emulsionen über das Phasenvolumenverhältnis führt in der Regel zu zähflüssigen Formulierungen.

Emulsionen von „flüssiger" (= fließfähiger) Konsistenz finden in der Kosmetik beispielsweise als Pflege-, Reinigungs-, Gesichts- oder Handlotion Verwendung. Sie haben in der Regel eine Viskosität von etwa 2000 mPa • s bis zu etwa 10 000 mPa • s. Der Stabilität von fließfähigen Emulsionen ist besondere Aufmerksamkeit zu widmen, da die erheblich größere Beweglichkeit der Teilchen eine schnellere Koaleszenz fördert.

Auch diese flüssigen Emulsionen des Standes der Technik sind - da auch sie i. a. Verdickungsmittel enthalten - gegenüber höheren Elektrolytkonzentrationen nicht stabil, was sich in einer Phasentrennung äußert. Es ist aber häufig wünschenswert, bestimmte Elektrolyte, wie beispielsweise wasserlösliche UV-Filter, einzusetzen, um deren sonstige physikalische, chemische bzw. physiologische Eigenschaften nutzen zu können. Zwar läßt sich in vielen Fällen durch geeignete Wahl des Emulgatorsystems in gewissem Maß Abhilfe schaffen, es treten dann aber ebensooft andere Nachteile auf.

Die angesprochenen Nachteile können beispielsweise darin liegen, daß vergleichsweise große Mengen an einem oder mehreren Emulgatoren erforderlich sind (z. B. 3 Gew.-% oder mehr). Da aber auch Emulgatoren - wie letztendlich jede chemische Substanz - im Einzelfall allergische oder auf Überempfindlichkeit des Anwenders beruhende Reaktionen hervorrufen können (obwohl die Verwendung der üblichen kosmetischen Emulgatoren i. a. natürlich völlig unbedenklich ist) ist es wünschenswert, den Emulgatorgehalt einer kosmetischen oder dermatologischen Formulierung so klein wie möglich zu halten.

Emulsionen mit einer sehr geringen Viskosität (dünnflüssige bzw. sprühbare Emulsionen) sind nach dem oben gesagten bislang - wenn überhaupt - nur sehr aufwendig zu formulieren. Dementsprechend ist das Angebot an derartigen Formulierungen äußerst gering. Gleichwohl könnten derartige Formulierungen dem Verbraucher bisher nicht gekannte kosmetische Leistungen bieten.

Zwar beschreibt die Europäische Patentschrift 667 144 kosmetische Sonnenschutzmittel, welche Öl-in-Wasser-Emulsionen darstellen und anorganische Nanopigmente auf der Basis von Metalloxiden als Lichtschutzmittel enthalten, wobei die Formulierungen auch sprühbar sein können. Diese Zubereitungen stellen sogenannte PIT-Emulsionen dar, welche durch Phasenumkehr hergestellt werden und daher besonders feindispers sind. Allerdings konnte diese Schrift nicht den Weg zur vorliegenden Erfindung weisen.

Überhaupt haben dünnflüssige Zubereitungen des Standes der Technik häufig den Nachteil, daß sie auf einen engen Anwendungsbereich oder eine eingeschränkte Rohstoffauswahl begrenzt sind. Auch die Einarbeitung höherer Konzentrationen an polaren Ölkomponenten bereitet häufig Schwierigkeiten. Es ist aber gegebenenfalls wünschenswert, hohe Mengen polarer Ölkomponenten in eine Formulierung einzuarbeiten, beispielsweise um feste UV-Filtersubstanzen lösen und so einen hohen Lichtschutzfaktor erreichen zu können.

Eine Aufgabe der vorliegenden Erfindung war es, Zubereitungen vom Typ Öl-in-Wasser herzustellen, welche eine sehr geringe Viskosität haben und nicht die Nachteile des Standes der Technik aufweisen. Eine weitere Aufgabe der Erfindung war, Lösungswege zu kosmetischen oder dermatologischen, möglichst dünnflüssigen O/W-Emulsionen aufzudecken, welche gegenüber erhöhten Elektrolytkonzentrationen stabil sind und in die sich hohe Mengen an polaren Ölkomponenten einarbeiten lassen. Ferner war Aufgabe der Erfindung, ein Verfahren zur Stabilisierung von O/W-Formulierungen zu finden.

Erstaunlicherweise werden diese Aufgaben gelöst durch

dünnflüssige kosmetische oder dermatologische Zubereitungen vom Typ Öl-in-Wasser, welche eine Ölphase, in welche hydrophobe und/oder amphiphile Feststoffe eingearbeitet sind, und eine Wasserphase enthalten, wobei die Dichtedifferenz zwischen der Ölphase und der Wasserphase (bestimmbar mit einem rechnenden digitalen Dichtemesser vom Typ DMA 45 der Firma chempro/PA bei 25 °C) nicht größer als 0,01 g • $cm^{-3}$ ist

sowie gewünschtenfalls übliche kosmetische oder dermatologische Hilfs-, Zusatz- und/oder Wirkstoffe enthaltend.

Gegenstand der Erfindung ist ferner ein

Verfahren zur Stabilisierung von O/W-Formulierungen, dadurch gekennzeichnet, daß die Dichte der Ölphase durch Zugabe von hydrophoben und/oder amphiphilen Feststoffen der Dichte der Wasserphase derart angeglichen wird, daß die Dichtedifferenz der beiden Phasen nicht größer als 0,01 g • $cm^{-3}$ ist.

Auch nach diesem Verfahren erhältliche O/W-Formulierungen sind Gegenstand der vorliegenden Erfindung.

Die erfindungsgemäßen Zubereitungen stellen in jeglicher Hinsicht überaus befriedigende Präparate dar, welche nicht auf eine eingeschränkte Rohstoffauswahl begrenzt sind. Dementsprechend eignen sie sich ganz besonders, um als Grundlage für Zubereitungsformen mit vielfältigen Anwendungszwecken zu dienen. Die erfindungsgemäßen Zubereitungen haben eine ausgezeichnete Stabilität gegenüber einem Zerfall in Öl- und Wasserphasen und zeigen sehr gute sensorische Eigenschaften, wie beispielsweise die Verteilbarkeit auf der Haut oder das Einzugsvermögen in die Haut.

Es war insbesondere überraschend, daß die erfindungsgemäßen Zubereitungen auch ohne Zusatz weitere Stabilisatoren - wie beispielsweise Konsistenzgeber, Verdickungsmittel oder Schutzkolloide usw. - außerordentlich stabil sind und daß sich beispielsweise größere Mengen polarer Ölkomponenten problemlos einarbeiten lassen.

Die erfindungsgemäßen Zubereitungen stellen in jeder Hinsicht eine Bereicherung des Standes der Technik in bezug auf dünnflüssige O/W-Emulsionen dar.

Ferner sind nach dem erfindungsgemäßen Verfahren in erstaunlich einfacher Weise sehr stabile O/W-Formulierungen, beispielsweise sprühbare Formulierungen mit einem hohen Lichtschutzfaktor erhältlich.

Es ist erfindungsgemäß besonders vorteilhaft, wenn die Zubereitungen deutlich weniger als 1 Gew.-% (bezogen auf das Gesamtgewicht der Zubereitungen) eines oder mehrerer Emulgatoren enthalten. Ganz besonders bevorzugt sind erfindungsgemäße Zubereitungen, welche - bezogen auf das Gesamtgewicht der Zubereitungen - weniger als 0,5 Gew.-% eines oder mehrerer Emulgatoren enthalten bzw. welche sogar gänzlich frei von Emulgatoren sind.

Es ist ferner vorteilhaft, wenn der mittlere Durchmesser der Öltröpfchen der erfindungsgemäßen Formulierungen kleiner als 50 μm ist. Bevorzugt im Sinne der vorliegenden Erfindung sind Formulierungen, deren Gesamtdichte größer ist als 0,9 g • $cm^{-3}$, insbesondere größer als 0,95 g • $cm^{-3}$.

Es kann ferner vorteilhaft sein, wenn die erfindungsgemäßen O/W-Formulierungen, obwohl es nicht notwendig ist, auch Stabilisatoren enthalten, welche vorteilhaft aus der Gruppe der Verdickungsmittel gewählt werden. Es ist vorteilhaft, den Gehalt an dem oder den Verdickungsmitteln aus dem Bereich 0,05 Gew.-% bis 0,15 Gew.-% zu wählen, bezogen auf das Gesamtgewicht der Zubereitungen.

**Feststoffe**

Vorteilhafte amphiphile Feststoffe im Sinne der vorliegenden Erfindung sind beispielsweise modifizierte Schichtsilikate.

Silikate sind Salze und Ester (Kieselsäureester) der Orthokieselsäure [$Si(OH)_4$] und deren Kondensationsprodukte. Die Silikate sind nicht nur die artenreichste Klasse der Mineralien, sondern auch geologisch und technisch außerordentlich wichtig. Über 80 % der Erdkruste bestehen aus Silikaten. Schichtsilikate (Phyllosilikate, Blattsilikate) sind (idealerweise) Silikat-Strukturen mit zweidimensional unendlichen Schichten aus $[SiO_4]^{4-}$-Tetraedem, wobei jedes Tetraeder über 3 Brücken-Sauerstoffe mit Nachbar-Tetraedern verbunden ist.

Chemische Formeln lassen sich für Schichtsilikate nur angenähert aufstellen, da sie ein großes Ionenaustausch-Vermögen besitzen und Silizium gegen Aluminium und dieses wiederum gegen Magnesium, $Fe^{2+}$, $Fe^{3+}$, Zn und dergleichen ausgetauscht werden kann. Die daraus möglicherweise resultierende negative Ladung der Schichten wird in der Regel durch Kationen, insbesondere durch $Na^{+}$ und $Ca^{2+}$ in Zwischenschicht-Positionen ausgeglichen.

Schichtsilikate können durch reversible Einlagerung von Wasser (in der 2- bis 7-fachen Menge) und anderen Substanzen wie z. B. Alkoholen, Glykolen und dergleichen mehr aufquellen. Ihre Verwendung als Verdickungsmittel in kosmetischen Mitteln ist dementsprechend an sich bekannt. Allerdings konnte der Stand der Technik nicht den Weg zur vorliegenden Erfindung weisen.

Vorteilhafte Schichtsilikate im Sinne der vorliegenden Erfindung sind beispielsweise solche, deren größte Ausdehnungsrichtung im unmodifizierten und ungequollenen Zustand im Mittel eine Länge von weniger als 10 μm hat. Beispielsweise können die mittleren Ausdehnungen der verwendeten modifizierten Schichtsilikatpartikel bei 1000 nm x 100 nm x 1 nm und darunter liegen. Die effektive Größe der modifizierten Schichtsilikatpartikel in einer kosmetischen oder dermatologischen Formulierung hängt selbstverständlich von der Menge an eingelagerten Substanzen ab.

Vorteilhafte modifizierte Schichtsilikate im Sinne der vorliegenden Erfindung sind beispielsweise modifizierte Smektite (Smectite).

Smektite sind stets sehr feinkörnige (meist < 2 mm), überwiegend als lamellenförmige, moosartige oder kugelförmige Aggregate vorkommende Dreischicht-Tonminerale (2:1-Schichtsilikate), in denen eine zentrale Schicht aus oktaedrisch koordinierten Kationen sandwichartig von 2 Schichten aus [$(Si,Al)O_4$]-Tetraedern umgeben ist. Smektite werden idealisiert durch die folgende Strukturformel beschrieben, worin weiß ausgefüllte Kreise Silizium- und/oder Aluminiumatome, hellgrau ausgefüllte Kreise Sauerstoffatome, dunkelgrau ausgefüllte Kreise Wasserstoffatome und schwarz

ausgefüllte Kreise Aluminium-, Magnesium-, Eisenatome und/oder weitere Austauschkationen darstellen:

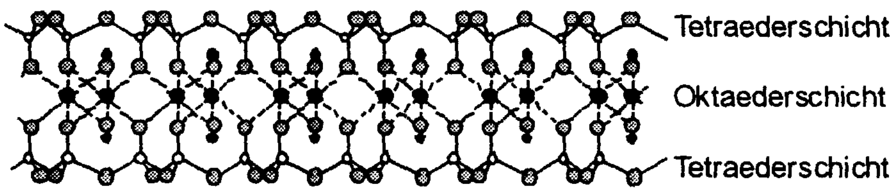


Vorteilhafte modifizierte Smektite sind z. B. modifizierte Montmorillonite. Montmorillonite werden durch die angenäherte chemische Formel $Al_2[(OH)_2/Si_4O_{10}] \cdot n\,H_2O$ bzw. $Al_2O_3 \cdot 4\,SiO_2 \cdot H_2O \cdot n\,H_2O$ beschrieben und stellen zu den dioktaedrischen Smektiten gehörende Tonmineralien dar.

Besonders vorteilhaft im Sinne der vorliegenden Erfindung sind ferner beispielsweise modifizierte Hektorite. Hektorite gehören zu den Smektiten und haben die angenäherte chemische Formel $M^{+}_{0,3}(Mg_{2,7}Li_{0,3})[Si_4O_{10}(OH)_2]$, worin $M^+$ meist $Na^+$ darstellt.

Vorteilhaft im Sinne der vorliegenden Erfindung sind ferner modifizierte Bentonite. Bentonite sind Tone und Gesteine, die Smektite, vor allem Montmorillonit, als Hauptminerale enthalten. Die „Roh"-Bentonite sind entweder Calcium-Bentonite (in Großbritannien als Fuller-Erden bezeichnet) oder Natrium-Bentonite (auch: Wyoming-Bentonite).

Modifizierte Schichtsilikate im Sinne der vorliegenden Erfindung sind Schichtsilikate, insbesondere die bereits genannten Schichtsilikattypen, deren Organophilie (auch: Lipophilie) - beispielsweise durch Umsetzung mit quarternären Ammonium-Verbindungen - erhöht wurde. Solche Schichtsilikate werden auch als organophile Schichtsilikate bezeichnet.

Besonders vorteilhaft im Sinne der vorliegenden Erfindung sind sogenannte Bentone, d. h. organische Derivate von Montmorilloniten (bzw. Bentoniten) und/oder Hectoriten, die durch Ionenaustausch-Reaktionen mit Alkylammonium-Basen hergestellt werden.

Vorteilhafte modifizierte Schichtsilikate im Sinne der vorliegenden Erfindung sind beispielsweise durch Umsetzung von Schichtsilikaten mit Quatemium-18 erhältlich. Quaternium-18 ist eine Mischung von quaternären Ammoniumchloridsalzen, welche durch die folgende Strukturformel beschrieben werden:

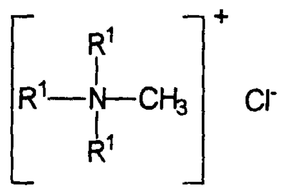

worin
die Reste $R^1$ unabhängig voneinander gewählt werden aus der Gruppe Methyl und hydrierte Talgreste mit einer Kettenlänge von 12 bis 20 Kohlenstoffatomen.

Erfindungsgemäß besonders bevorzugt sind Stearalkoniumhektorit, ein Reaktionsprodukt aus Hektorit und Stearalkoniumchlorid (Benzyldimethylstearylammoniumchlorid), und Quaternium-18 Hektorit, ein Reaktionsprodukt aus Hektorit und Quaternium-18, welche z. B. unter den Handeisbezeichnungen Bentone 27 und Bentone 38 bei Nordmann & Rassmann erhältlich sind.

Vorteilhafte hydrophobe und/oder amphiphile Feststoffe sind ferner röntgenamorphe Oxidpigmente. Röntgenamorphe Oxidpigmente sind Metalloxide oder Halbmetalloxide, welche bei Röntgenbeugungsexperimenten keine oder keine erkenntliche Kristallstruktur erkennen lassen. Oftmals sind solche Pigmente durch Flammenreaktion erhältlich, beispielsweise dadurch, daß ein Metall- oder Halbmetallhalogenid mit Wasserstoff und Luft (oder reinem Sauerstoff) in einer Flamme umgesetzt wird.

Bevorzugte röntgenamorphe Oxidpigmente sind Siliciumoxide des Typs Aerosil® (CAS-Nr. 7631-86-9). Aerosile®, die bei der DEGUSSA AG/Frankfurt erhältlich sind, zeichnen sich durch eine geringe Partikelgröße (z.B. zwischen 5 und 40 nm) aus, wobei die Partikel als kugelförmige Teilchen sehr einheitlicher Abmessung anzusehen sind. Makroskopisch sind Aerosile® als lockere, weiße Pulver erkenntlich.

Vorteilhafte Aerosil®-Typen sind beispielsweise Aerosil® OX50, Aerosil® 130, Aerosil® 150, Aerosil® 200,

Aerosil® 300, Aerosil® 380, Aerosil® MOX 80, Aerosil® MOX 170, Aerosil® COK 84, Aerosil® R 202, Aerosil® R 805, Aerosil® R 812, Aerosil® R 974, Aerosil® R976.

Weitere vorteilhafte hydrophobe und/oder amphiphile Feststoffe sind beispielsweise mikronisierte, anorganische Pigmente, die gewählt werden aus der Gruppe der amphiphilen und/oder hydrophoben Metalloxide, insbesondere aus der Gruppe Titandioxid, Zinkoxid und Siliciumdioxid, wobei die Metalloxide sowohl einzeln als auch im Gemisch vorliegen können. Dabei ist es im wesentlichen unerheblich, in welcher der gegebenenfalls natürlich vorkommenden Modifikationen die verwendeten amphiphilen Metalloxide vorliegen.

Vorteilhaft ist es, den mittleren Partikeldurchmesser der verwendeten Pigmente zwischen 1 nm und 200 nm, besonders vorteilhaft zwischen 5 nm und 100 nm zu wählen.

Erfindungsgemäß können die kosmetischen und dermatologischen Zubereitungen auch hydrophobe anorganische Mikropigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid.

Vorteilhaft im Sinne der vorliegenden Erfindung ist es auch, nahezu reine Pigmentpartikel zu verwenden, insbesondere solche, welche auch als Farbstoff in der Lebensmittelindustrie verwendet werden können. Vorteilhaft sind beispielsweise die bei der Firma Merck erhältlichen Zinkoxid-Pigmente sowie solche, die unter den Handelsbezeichnungen Zinkoxid neutral bei Haarmann & Reimer oder NanoX von der Harcros Chemical Group erhältlich sind.

Vorteilhafte nahezu reine Pigmentpartikel sind im Sinne der vorliegenden Erfindung ferner die im folgenden aufgelisteten Bornitride:

| Handelsname | erhältlich bei |
|---|---|
| Boron Nitride Powder | Advanced Ceramics |
| Boron Nitride Powder | Sintec Keramik |
| Ceram Blanche | Kawasaki |
| HCST Boron Nitride | Stark |
| Très BN® | Carborundum |
| Wacker-Bornitrid BNP | Wacker-Chemie |

Vorteilhaft ist es, den mittleren Partikeldurchmesser der verwendeten Bornitridpartikel kleiner als 20 µm, besonders vorteilhaft kleiner als 15 µm zu wählen.

Erfindungsgemäß vorteilhaft sind ferner Feststoffe, die oberflächlich wasserabweisend behandelt („gecoatet") sind, wobei ein hydrophober und/oder amphiphiler Charakter dieser Feststoffe gebildet werden bzw. erhalten bleiben soll. Diese Oberflächenbehandlung kann darin bestehen, daß die Feststoffe nach an sich bekannten Verfahren mit einer dünnen hydrophoben Schicht versehen werden.

Ein solches Verfahren, das im folgenden am Beispiel von Titandioxid beschrieben wird, besteht beispielsweise dann, daß die hydrophobe Oberflächenschicht nach einer Reaktion gemäß $n\ TiO_2 + m\ (RO)_3\ Si\text{-}R' \rightarrow n\ TiO_2\ (\text{oberfl.})$ erzeugt wird. n und m sind dabei nach Belieben einzusetzende stöchiometrische Parameter, R und R' die gewünschten organischen Reste. Besonders vorteilhaft sind $TiO_2$-Pigmente, beispielsweise die mit Aluminiumstearat beschichteten, unter der Handelsbezeichnung MT 100 T bei der Firma TAYCA erhältlichen.

Eine weitere vorteilhafte Beschichtung besteht aus Aluminiumhydroxid bzw. Aluminiumoxidhydrat (auch: Alumina, CAS-Nr.: 1333-84-2), auf das anschließend Stearinsäure aufgebracht wird. Es ist darüber hinaus auch bevorzugt, andere hydrophobe Beschichtungen auf mit Alumina vorbehandelte Metalloxidpartikel aufzubringen, wie z. B. Polyorganosiloxane. Vorteilhafte mit Alumina und Stearinsäure beschichtete, hydrophobe Titandioxidpigmente sind beispielsweise unter der Handelsbezeichnung UV Titan M160 bei Kemira erhältlich.

Eine weitere vorteilhafte Beschichtung der erfindungsgemäßen Feststoffe besteht aus Dimethylpolysiloxan (auch: Dimethicone), einem Gemisch vollmethylierter, linearer Siloxanpolymere, die endständig mit Trimethylsiloxy-Einheiten blockiert sind. Besonders vorteilhaft im Sinne der vorliegenden Erfindung sind Zinkoxid-Pigmente und Bornitridpartikel, die auf diese Weise beschichtet werden. Vorteilhaft sind beispielsweise die bei der Firma Carborundum unter der Handelsbezeichnung Très BN® UHP 1106 erhältlichen, mit Dimethicone behandelten Bornitridpartikel.

Vorteilhaft ist ferner eine Beschichtung mit Polymethylhydrogensiloxan, einem linearen Polysiloxan, welches

auch als Methicone bezeichnet wird. Vorteilhafte, mit Methicone behandelte Bornitridpartikel sind beispielsweise die bei der Firma Carborundum unter der Handelsbezeichnung Très BN® UHP 1107 erhältlichen.

Vorteilhaft ist ferner, wenn die erfindungsgemäßen Feststoffe mit einem Gemisch aus Dimethylpolysiloxan, insbesondere Dimethylpolysiloxan mit einer durchschnittlichen Kettenlänge von 200 bis 350 Dimethylsiloxan-Einheiten, und Silicagel beschichtet sind, welches auch als Simethicone bezeichnet wird. Es ist insbesondere von Vorteil, wenn die anorganischen Pigmente zusätzlich mit Aluminiumhydroxid bzw. Aluminiumoxidhydrat (auch: Alumina, CAS-Nr.: 1333-84-2) beschichtet sind. Besonders vorteilhaft sind Titandioxide, die mit Simethicone und Alumina beschichtet sind, wobei die Beschichtung auch Wasser enthalten kann. Ein Beispiel hierfür ist das unter dem Handelsnamen Eusolex T2000 bei der Firma Merck erhältliche Titandioxid.

Erfindungsgemäße vorteilhaft sind ferner beispielsweise Titandioxidpigmente, die mit Octylsilanol beschichtet sind, und/oder Siliciumdioxidpartikel, die oberflächlich wasserabweisend behandelt sind. Bevorzugt sind beispielsweise sphärische Polyalkylsilsesquioxan-Partikel wie sie in der Europäischen Offenlegungsschrift 0 686 391 erwähnt werden. Solche Polyalkylsilsesquioxan-Partikel sind beispielsweise unter den Handelsbezeichnungen Aerosil R972 und Aerosil 200V bei der Firma Degussa erhältlich.

Vorteilhaft im Sinne der vorliegenden Erfindung sind ferner Mischungen verschiedener anorganischer, amphiphiler Pigmenttypen sowohl innerhalb eines Kristalls, beispielsweise als Eisenmischoxid oder Talkum (Magnesiumsilicat), als auch durch Mischung mehrerer Metalloxidtypen innerhalb einer Zubereitung. Besonders vorteilhaft sind Magnesiumsilicate, beispielsweise die unter der Handelsbezeichnung Talkum Micron bei der Firma Grolmann erhältlichen.

Erfindungsgemäß vorteilhaft sind ferner Dispersionen von ultrafeinem Titandioxid in Ölen bzw. ölige Titandioxid-Suspensionen, z. B. Titandioxid in Caprylic-/Capric Triglycerid, einem Gemisch von Triglyceriden hauptsächlich der Caprylsäure [$CH_3(CH_2)_6COOH$] und der Caprinsäure [$CH_3(CH_2)_8COOH$]. Bevorzugt sind beispielsweise die unter der Handeisbezeichnung Tioveil TG bei der Firma Solaveil erhältlichen öligen Titandioxid-Suspensionen.

Vorteilhaft im Sinne der vorliegenden Erfindung sind ferner Glanz- oder Perlpigmente, insbesondere auch sogenannte Silber- und Goldglanzpigmente. Bevorzugt sind pulverförmige Pigmente oder Rizinusöldispersionen von Wismutoxychlorid und/oder Titandioxid sowie Wismutoxychlorid und/oder Titandioxid auf Glimmer. Solche Wismutoxychloride werden in unterschiedlichen Qualitäten von Merck Rona unter der Handelsbezeichnung Biron angeboten. Vorteilhaft ist beispielsweise Biron LF 2000. Besonders bevorzugt sind ferner die unter der Handelsbezeichnung Mica bei der Merck KGaA erhältlichen Perlpigmente, insbesondere Mica Black, welches eine Mischung aus Eisenoxid ($Fe_3O_4$), Glimmer und Titandioxid darstellt, und Mica M.

Vorteilhafte Feststoffe im Sinne der vorliegenden Erfindung sind ferner Pigmente, welche eine färbende Wirkung haben, beispielsweise die im folgenden aufgelisteten (in Klammern die Colour Index Nummern nach dem *Rowe Colour Index, 3. Auflage, Society of Dyers and Colourists, Bradford, England, 1971* und der Farbton):
Pigment Green (10006, grün), Pigment Yellow 1 (11680, gelb), Pigment Yellow 3 (11710, gelb), Pigment Orange 1 (11725, orange), Pigment Red 3 (12120, rot), Pigment Red 112 (12370, rot), Pigment Red 7 (12420, rot), Pigment Brown 1 (12480, braun), Pigment Yellow 16 (20040, gelb), Pigment Yellow 13 (21100, gelb), Pigment Yellow 83 (21108, gelb), Pigment Violet 23 (51319, violett), Pigment Red 122 (73915, rot), Pigment Blue 16 (74100, blau), Aluminium (77000, weiß), Tonerdehydrat (77002, weiß), Pigment Red 101 und 102 (77015, rot), Bariumsulfat (77120, weiß), Kohlenstoff (77266, schwarz), Pigment Black 9 (77267, schwarz), Carbo medicinalis vegetabilis (77268:1, schwarz), Pigment Blue 28 und Pigment Green 14 (77346, grün), Pigment Metal 2 (77400, braun), Gold (77480, braun), Eisenoxide und -hydroxide (77489, orange), Eisenoxid (77491, rot), Eisenoxidhydrat (77492, gelb), Eisenoxid (77499, schwarz), Mischungen aus Eisen(II)- und Eisen(III)-hexacyanoferrat (77510, blau), Pigment White 18 (77713, weiß) und Silber (77820, weiß).

Weitere vorteilhafte hydrophobe und/oder amphiphile Feststoffe sind mikrofeine Polymerpartikel, welche in der Zubereitung in Form von Feststoffen vorliegen. Günstig im Sinne der vorliegenden Erfindung sind beispielsweise Polycarbonate, Polyether, Polyethylen, Polypropylen, Polyvinylchlorid, Polystyrol, Polyamide, Polyacrylate und dergleichen mehr.

Vorteilhaft sind beispielsweise mikrofeine Polyamid-Partikel, insbesondere die unter der Handelsbezeichnung SP-500 bei der Firma TORAY erhältlichen. Ferner vorteilhaft sind Polyamid 6- (auch: Nylon 6) bzw. Polyamid 12- (auch: Nylon 12) Partikel. Polyamid 6 ist das aus ε-Aminocapronsäure (6-Aminohexansäure) oder ε-Caprolactam aufgebaute Polyamid [Poly(ε-caprolactam)], und Polyamid 12 ist ein Poly(ε-laurinlactam) aus ε-Laurinlactam. Vorteilhaft im Sinne der vorliegenden Erfindung sind beispielsweise Orgasol® 1002 (Polyamid 6) und Orgasol® 2002 (Polyamid 12) von der Firma ELF ATOCHEM.

Weitere vorteilhafte mikrofeine Polymerpartikel sind mikrofeine Polymethacrylate. Solche Partikel sind beispielsweise unter der Handelsbezeichnung POLYTRAP® bei der Firma DOW CHEMICAL erhältlich.

Es ist insbesondere vorteilhaft, wenngleich nicht zwingend, wenn die mikrofeinen Polymerpartikel oberflächlich beschichtet sind. Diese Oberflächenbehandlung kann dann bestehen, daß die Polymerpartikel nach an sich bekannten Verfahren mit einer dünnen hydrophilen Schicht versehen werden. Vorteilhafte Beschichtungen bestehen

beispielsweise aus Titandioxid ($TiO_2$), Zirkoniumdioxid ($ZrO_2$) oder auch weiteren Polymeren, wie beispielsweise Polymethylmethacrylat. Besonders vorteilhafte mikrofeine Polymerpartikel im Sinne der vorliegenden Erfindung sind beispielsweise die nach dem in der US-Patentschrift 4,898,913 beschriebenen Verfahren zur hydrophilen Beschichtung hydrophober Polymerpartikel erhältlichen.

Der mittlere Partikeldurchmesser der verwendeten mikrofeinen Polymerpartikel wird vorzugsweise kleiner als 100 µm, besonders vorteilhaft kleiner als 50 µm gewählt. Dabei ist es im wesentlichen unerheblich, in welcher Form (Plättchen, Stäbchen, Kügelchen etc.) die verwendeten Polymerpartikel vorliegen.

Weitere bevorzugte hydrophobe und/oder amphiphile Feststoffe sind amphiphile modifizierte Polysaccharide, welche keine verdickenden Eigenschaften zeigen.

Solche amphiphilen Polysaccharide sind beispielsweise durch Umsetzung von Stärke mit mono-, bi- oder polyfunktionellen Reagenzien bzw. Oxidations-Mitteln in weitgehend polymeranalog verlaufenden Reaktionen erhältlich.

Diese Reaktionen basieren im wesentlichen auf Umwandlungen der Hydroxy-Gruppen der Polyglucane durch Veretherung, Veresterung oder selektive Oxidation. Dabei entstehen z. B. sogenannte Stärkeether und Stärkeester der allgemeinen Strukturformel

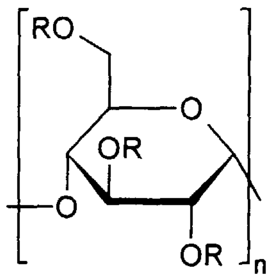

**Strukturformel I**

worin R beispielsweise ein Wasserstoff und/oder einen Alkyl- und/oder Aralkylrest (im Fall der Stärkeether) oder ein Wasserstoff und/oder einen organischen und/oder anorganischen Saure-Rest (im Fall der Stärkeester) darstellen kann. Stärkeether und Stärkeester sind vorteilhaft im Sinne der vorliegenden Erfindung.

Besonders vorteilhaft ist es, Stärkeether einzusetzen, z. 8. solche, die durch Veretherung von Stärke mit Tetramethylolacetylendiharnstoff erhältlich sind und welche als Amylum non mucilaginosum (nicht quellende Stärke) bezeichnet werden.

Insbesondere vorteilhaft sind Stärkeester und/oder deren Salze, beispielsweise Natrium- und/oder Aluminiumsalze niedrigsubstituierter Halbester der Stärke, insbesondere Natrium Stärke n-Octenylsuccinat der Strukturforrnel I, worin R sich durch die folgende Struktur auszeichnet

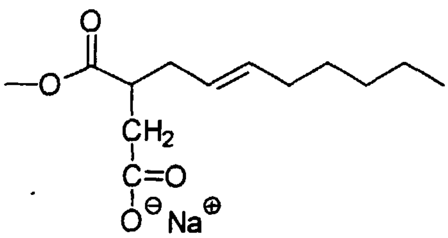

und welches z. B. unter der Handeisbezeichnung Amiogum® 23 bei der Firma CERESTAR erhältlich ist sowie Aluminium Stärke Octenylsuccinat, insbesondere die unter den Handelsbezeichnungen Dry Flo® Elite LL und Dry Flo® PC bei der Firma CERESTAR erhältlichen.

Ferner vorteilhaft ist Distärkephosphat (INCI: Distarch Phosphate), das durch Kreuzvernetzung von Stärke mit Natriummetaphosphat entsteht und unter der Handelsbezeichnung Mais OP bei der Firma chemag erhältlich ist.

Vorteilhaft ist es, den mittleren Partikeldurchmesser der modifizierten Polysaccharide kleiner als 20 µm, besonders vorteilhaft kleiner als 15 µm zu wählen.

Die Liste der genannten modifizierten Polysaccharide, die mit den modifizierten Schichtsilikaten kombiniert

werden können, soll selbstverständlich nicht limitierend sein. Modifizierte Polysaccharide, die im Sinne der vorliegenden Erfindung vorteilhafte Feststoffe darstellen, sind auf zahlreichen, an sich bekannten Wegen, sowohl chemischer als auch physikalischer Natur erhältlich. Zur Herstellung solcher Polysaccharide sind auch neue Wege prinzipiell denkbar. Wesentlich dabei ist, daß die modifizierten Polysaccharide hydrophobe und/oder amphiphile Eigenschaften zeigen und daß sie nicht verdickend wirken.

Vorteilhaft in allen vorgenannten Fällen ist es, die Gesamtkonzentration aller Pigmente größer als 0,05 Gew.-%, besonders vorteilhaft zwischen 0,05 Gew.-% und 30 Gew.-% bezogen auf das Gesamtgewicht der Zubereitungen zu wählen, wobei der Gesamtgehalt an einem oder mehreren hydrophoben und/oder amphiphilen Feststoffen so zu wählen ist, daß die Dichtedifferenz zwischen der Ölphase und der Wasserphase (bestimmbar mit einem rechnenden digitalen Dichtemesser vom Typ DMA 45 der Firma chempro/PA bei 25 °C) nicht größer als 0,01 g • $cm^{-3}$ ist.

Die hydrophoben und/oder amphiphilen Feststoffe werden in die Ölphase der Formulierungen eingearbeitet. Die jeweils einzusetzenden Feststoffmengen können in Abhängigkeit von der Art des jeweiligen Produktes vom Fachmann ohne erfinderisches Zutun durch einfaches Ausprobieren leicht ermittelt werden.

**Ölphase**

Die Ölphase der erfindungsgemäßen O/W-Emulsionen wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z.B. Caprylic/Caprictriglycerid, Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodecylmyristat, Cetearylisononanoat, Isopropylmyristat, Isopropylpalmitat, Isopropylstearat, Isopropyloleat, n-Butylstearat, n-Hexyllaurat, ri-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleyterucat, Erucyloleat, Erucylerucat, Tridecylstearat, Tridecyltrimellitat, sowie synthetische, halbsynthetische und natürliche Gemische solcher Ester, wie z.B. Jojobaöl.

Ferner kann die Ölphase vorteilhaft gewählt werden aus der Gruppe der Dialkylether, vorteilhaft ist z. B. Dicaprylylether.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol CaprylatiCaprat, $C_{12-13}$-Alkyllactat, Di-$C_{12/13}$-Atkyltartrat, Triisostearin, Dipentaerythrityl Hexacaprylat/Hexacaprat, Propylenglykolmonoisostearat, Tricaprylin, Dimethylisosorbid zu wählen. Es ist insbesondere vorteilhaft, wenn die Ölphase der erfindungsgemäßen O/W-Emulsionen einen Gehalt an $C_{12-15}$-Alkylbenzoat aufweist oder vollständig aus diesem besteht.

Auch beliebige Abmischungen solcher Öl- und Wachskomponenten sind vorteilhaft im Sinne der vorliegenden Erfindung einzusetzen.

Ferner kann die Ölphase der erfindungsgemäßen O/W-Emulsionen ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Erfindungsgemäß vorteilhaft sind ferner z. B. natürliche Wachse tierischen und pflanzlichen Ursprungs, wie beispielsweise Bienenwachs, Chinawachs, Hummelwachs und andere Insektenwachse, insbesondere die nachstehend genannten.

Bienenwachs z. B. ist ein Ausscheidungsprodukt aus Drüsen der Honigbienen, das diese zum Bauen der

Honigwaben verwenden. Gelbes (Cera flava), braunes oder rotes sogenanntes Rohwachs ist beispielsweise erhältlich, indem man die vom Honig durch Ausschleudem befreiten Waben schmilzt, die Schmelze von festen Verunreinigungen trennt und das so erhaltene Rohwachs erstarren läßt. Das Rohwachs kann durch Behandlung mit Oxidationsmitteln vollkommen weiß gebleicht werden (Gera alba).

Bienenwachs besteht aus dem in Alkohol leichtlöslichen Cerin, einem Gemisch aus Cerotinsäure $CH_3(CH_2)_{24}COOH$ und Melissinsäure $CH_3(CH_2)_{28}COOH$ sowie aus einem Myricin genannten Ester-Gemisch aus ca. 70 Estern von $C_{16}$- bis $C_{36}$-Säuren und $C_{24}$-bis $C_{36}$-Alkoholen. Als wesentliche Bestandteile von Bienenwachs finden sich Myricylpalmitat, Myricylcerotinat und Paraffin.

Auch andere Insektenwachse wie beispielsweise Hummelwachs, Schellackwachs oder Chinawachs sind im wesentlichen Mischungen verschiedener Ester. Chinawachs z. B. wird in China und Japan von der auf der chinesischen Esche lebenden Wachsschildlaus (Coccus ceriferus) und den Schildlausarten Ceroplastes ceriferus und Encerus pela abgeschieden bzw. erzeugt. Es wird von den Bäumen abgekratzt und durch Umschmelzen in kochendem Wasser gereinigt. Hauptbestandteil des Chinawachses ist der Cerotinsäureester des Cerylalkohols.

Schellackwachs wird aus Lac gewonnen, dem Sekret der weiblichen Lackschildläuse (Kerria lacca), welche in riesigen Kolonien (Lac ist abgeleitet von dem Hindhi-Wort „Lakh" für 100 000) auf Bäumen und Sträuchern im südasiatischen Raum (Indien, Burma, Südchina) leben. Das durch Läsemittel-Extraktion zugängliche Schellackwachs enthält als wesentliche Bestandteile Myricylalkohol, Melissinsäure und andere Wachsalkohole und -säuren bzw. deren Ester.

Auch Pflanzenwachse sind vorteilhaft im Sinne der vorliegenden Erfindung. Vorzugsweise verwendbar sind Cuticularwachse niederer und höherer Pflanzen, Algen, Flechten, Moose und Pilze, wie beispielsweise Candelillawachs, Carnaubawachs, Japanwachs, Espartograswachs, Korkwachs, Reiswachs, Zuckerrohrwachs, Fruchtwachse, z. B. Apfelwachs, Blütenwachse, Blattwachse von Nadelhölzern, Kaffeewachs, Flachswachs, Sesamwachs, Jojobaöl und dergleichen mehr.

Candelillawachse z. B. sind bräunliche bis gelblichbraune, harte wachsartige Massen, welche in lipophilen Läsemitteln löslich sind. Candelillawachs enthält ungeradzahlige ahphatische Kohlenwasserstoffe (ca. 42 %) Ester (ca. 39 %), Wachssäuren und Wachsalkohole. Gewonnen werden kann es beispielsweise aus den zerkleinerten, fleischigen Blättern einer stachellosen Wolfsmilchart (Euphorbia cerifera) durch Auskochen mit wäßriger Schwefelsäure.

Camaubawachs ist eine gelbliche, grünliche oder dunkelgraue Masse, welche in verschiedenen durch Auslese gewonnenen Qualitäten aus den Blättern der brasilianischen Fächerpalme Copernicia prunifera oder Carnaubapalme (Carnauba cerifera) gewonnen werden kann, indem beispielsweise der Wachsstaub von den angewelkten Wedeln gebürstet, geschmolzen, filtriert und nach dem Festwerden in Stücke gebrochen wird.

Carnaubawachs kann durch Bleichmittel aufgehellt werden. Es enthält ca. 85 % Ester, jeweils etwa 2-3 % freie Wachssäuren (Camauba-, Behen-, Lignocerin-, Melissin- und Cerotinsäure), langkettige Alkohole, Diole und gesättigte Kohlenwasserstoffe.

Japanwachs (auch: Japantalg oder Cera japonica) ist farbloses oder gelbliches, reines Pflanzenfett, das beispielsweise in Japan aus den Früchten eines baumförmigen Sumach-Gewächses (Rhus succedanea) durch Auskochen gewonnen werden kann. Hauptbestandteile des Japanwachses sind Palmitinsäureglycerinester sowie Ester der Japansäure (Heneicosandisäure, $C_{21}H_{40}O_4$), der Phellogensäure (Docosandisäure, $C_{22}H_{42}O_4$) und der Tricosandisäure ($C_{23}H_{44}O_4$).

Esparto-Wachs fällt als Nebenprodukt bei der Zellstoff- und Papierherstellung aus dem in Mittelmeerländern beheimateten Espartogras (Graminaceae) an. Es zu besteht zu ca. 15 bis 17 % aus Wachssäuren (z. B. Cerotin- und Melissinsäure), zu 20 bis 22 % aus Alkoholen und Kohlenwasserstoffen sowie zu 63 bis 65 % aus Estern.

Insbesondere vorteilhaft im Sinne der vorliegenden Erfindung sind beispielsweise die unter den Handelsbezeichnungen Permulgin 1550 und Permulgin 4002 bei KOSTER KEUNEN und die unter den Handeisbezeichnungen Schellack Wachs 7302 L und Candellila Wachs 2039 L bei der KAHL Wachsraffinerie erhältlichen natürlichen Wachse.

Erfindungsgemäß vorteilhaft sind ferner chemisch modifizierte Wachse und synthetische Wachse. Bevorzugte modifizierte Wachse sind beispielsweise Bienenwachsester, insbesondere die unter den Handelsnamen BW Ester BW 67, BW Ester BW 80 bei KOSTER KEUNEN erhältlichen Alkylbienenwachse.

Bevorzugte synthetische Wachse sind beispielsweise das unter der Handelsbezeichnung Bienenwachskomponente B 85 bei SCHLICKUM erhältliche sowie Wachse auf Silikonbasis wie z. B. Dialkoxydimethylpolysiloxane, welche sich durch die folgende Struktur auszeichnen

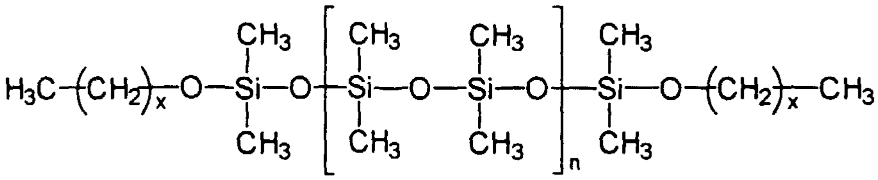

,

worin x eine Zahl zwischen 18 und 24 bedeutet. Insbesondere vorteilhaft ist das Behenoxy Dimethicon, für welches x aus der obigen Strukturformel 21 bedeutet und welches unter der Handeisbezeichnung Abil® Wax 2440 bei der Th. Goldschmidt AG erhältlich ist. Erfindungsgemäß bevorzugt ist ferner ein Wachs auf Silikonbasis, das unter der Handeisbezeichnung Siliconyl Beeswax bei KOSTER KEUNEN erhältlich ist.

Weitere vorteilhafte synthetische Wachse sind bestimmte Fettsäuren und/oder Fettsäuremischungen, beispielsweise $C_{16-36}$-Fettsäuren, insbesondere solche, die unter der Handelsbezeichnung Syncrowax AW1C bei Croda GmbH erhältlich sind.

Vorteilhaft im Sinne der vorliegenden Erfindung sind außerdem Esterwachse, die Ester aus

1. einer gesättigten und/oder ungesättigten, verzweigten und/oder unverzweigten Mono- und/oder Dicarbonsäure mit 12 bis 40 Kohlenstoffatomen und
2. einem gesättigten und/oder ungesättigten, verzweigten und/oder unverzweigten Alkohol mit 12 bis 40 Kohlenstoffatomen darstellen.

Insbesondere vorteilhaft sind Esterwachse, die gewählt werden aus der im folgenden aufgelisteten Gruppe:

| **Esterwachs** | **Handelsname** | **erhältlich bei** |
|---|---|---|
| Myristylmyristat | Cetiol MM | Henkel KGaA |
| Cetylpalmitat | Cutina CP | Henkel KGaA |
| $C_{14-34}$ Alkylstearat | Kesterwachs K 76 H | KOSTER KEUNEN |
| $C_{20-40}$ Dialkyldimerat | Kesterwachs K 80 D | KOSTER KEUNEN |
| Ditetracosanyldimerat | Kesterwachs K70D | KOSTER KEUNEN |
| $C_{16-38}$ Alkylhydroxystearoylstearat | Kesterwachs K80P | KOSTER KEUNEN |
| $C_{20-40}$ Alkylstearat | Kesterwachs K 82 | KOSTER KEUNEN |
| Hydroxystearylhydroxystearat | Elfacos C26 | AKZO NOBEL |

Weiterhin vorteilhaft sind Ester des Glykols, insbesondere Glykolester der Lignocerinsäure ($CH_3(CH_2)_{22}COOH$), der Cerotinsäure ($CH_3(CH_2)_{24}COOH$) und/oder der Montansäure ($CH_3(CH_2)_{26}COOH$). Ganz besonders vorteilhaft im Sinne der vorliegenden Erfindung sind Glykolester der Montansäure ($CH_3(CH_2)_{26}COOH$). Ein vorteilhaftes Glykolmontanat ist z.B. in einer Mischung mit Butylenglykolmontanat unter der Handelsbezeichnung Wax E Pharma bei der Firma Clariant erhältlich.

Es ist ferner vorteilhaft, die Wachskomponenten aus der Gruppe der Glyceride, insbesondere aus der Gruppe der Triglyceride zu wählen. Besonders vorteilhaft sind die im folgenden aufgelisteten Glyceride und Triglyceride:

| **Glycerid** | **Handelsname** | **erhältlich bei** |
|---|---|---|
| $C_{16-18}$ Triglycerid | Cremeol HF-52-SPC | Aarhus Oliefabrik |
| Glycerylhydroxystearat | Naturchem GMHS | Rahn |

(fortgesetzt)

| Glycerid | Handelsname | erhältlich bei |
|---|---|---|
| Hydrierte Coco-Glyceride | Softisan 100 | Hüls AG |
| Caprylic/Capric/Isostearic/Adipic Triglycerid | Softisan 649 | Dynamit Nobel |
| $C_{18-36}$ Triglycerid | Syncrowax HGLC | Croda GmbH |
| Glyceryltribehenat | Syncrowax HRC | Croda GmbH |
| Glyceryl-tri-(12-hydroxystearat) | Thixcin R | Rheox / NRC |
| Hydriertes Ricinusöl | Cutina HR | Henkel KGaA |
| $C_{16-24}$ Triglycerid | Cremeol HF-62-SPC | Aarhus Oliefabrik |

Besonders bevorzugt im Sinne der vorliegenden Erfindung ist auch Sheabutter, auch Karitéfett oder Galambutter genannt (CAS-Nr. 68920-03-6). Sheabutter ist das Fett der Samen bzw. Kerne der Familie der Sapotaceae angehörenden Pflanze Butyrospermum Parkii, das zu etwa 34 bis 45 Gew.-% aus festen Fettsäuren (vornehmlich Stearinsäure) und zu etwa 50 bis 60 Gew.-% aus flüssigen Fettsäuren (vornehmlich Ölsäure enthaltend) besteht.

**Kosmetische oder dermatologische Hilfs- und Zusatzstoffe**

Sollen die O/W-Formulierungen gemäß der vorliegenden Erfindung Emulgatoren enthalten, so sind solche Emulgatoren vorteilhaft zu verwenden, welche zur Herstellung von O/W-Emulsionen geeignet sind, wobei diese sowohl einzeln als auch in beliebigen Kombinationen miteinander vorliegen können.

Vorteilhaft werden der oder die Emulgatoren aus der Gruppe gewählt, die die folgenden Verbindungen umfaßt:

Polyglyceryl-2-Dipolyhydroxystearat, PEG-30-Dipolyhydroxystearat, Cetyldimethiconcopolyol, Glykoldistearat, Glykoldilaurat, Diethylenglykoldilaurat, Sorbitantrioleat, Glykololeat, Glyceryldilaurat, Sorbitantristearat, Propylenglykolstearat, Propylenglykollaurat, Propylenglykoldistearat, Sucrosedistearat, PEG-3 Castor Oil, Pentaerythritylmonostearat, Pentaerythritylsesquioleat, Glyceryloleat, Glycerylstearat, Glyceryldiisostearat, Pentaerythritylmonooleat, Sorbitansesquioleat, Isostearyldiglycerylsuccinat, Glycerylcaprat, Palm Glycerides, Cholesterol, Lanolin, Glyceryloleat (mit 40 % Monoester), Polyglyceryl-2-Sesquiisostearat, Polyglyceryl-2-Sesquioleat, PEG-20 Sorbitan Beeswax, Sorbitanoleat, Sorbitanisostearat, Trioleylphosphat, Glyceryl Stearate und Ceteareth-20 (Teginacid von Th. Goldschmidt), Sorbitanstearat, PEG-7 Hydrogenated Castor Oil, Steareth-2, Oleth-2, Cetyl Alcohol und Ceteareth-30 (Emulgator E 2209 von Th. Goldschmidt), PEG-5-Soyasterol, PEG-6 Sorbitan Beeswax, Ceteth-2, Glycerylstearat SE, Methylglucosesesquistearate, PEG-10 Hydrogenated Castor Oil, Oleth-3, Sorbitanpalmitat, PEG-22/Dodecylglykol Copolymer, Polyglyceryl-2-PEG-4-Stearat, Laneth-5, Ceteth-3, Laureth-3, Stearyl Alcohol und Steareth-7 und Steareth- 10 (Emulgator E-2155 von Th. Goldschmidt), Oleth-5, Sorbitanlaurat, Laureth-4, PEG-4-Laurat, Polysorbat 61, Polysorbat 81, Beheneth-10, Polysorbat 65, Polysorbat 80, Laneth-10, Triceteareth-4-Phosphat, Triceteareth-4 Phosphate und Sodium $C_{14-17}$ Alkyl Sec Sulfonat (Hostacerin CG von Hoechst), PEG-8 Stearat, Glycerylstearat und PEG-100 Stearate (Arlacel 165 von ICI), Polysorbat 85, Trilaureth-4-Phosphat, PEG-25-Glyceryltnoleat, Oleth-10, Steareth-10, Ceteth-10, PEG-35 Castor Oil, Sucrosestearat, PEG-8-Oleat, Trioleth-8-Phosphat, PEG-40-Sorbitanlanolat, PEG-15-Glycerylricinoleat, Choleth-24 und Ceteth-24 (Solulan C-24 von Amerchol), $C_{12-15}$ Pareth-12, PEG-20-Glycerylisostearat, PEG-40 Hydrogenated Castor Oil, PEG-16 Soya Sterol, PEG-20-Glyceryloleat, PEG-20-Stearat, Polysorbat 80, PEG-20-Methylglucosesesquistearat, PEG-30-Glycerylisostearat, PEG-20-Glyceryllaurat, Ceteth-20, Ceteareth-25, PEG-30-Stearat, PEG-30-Glycerylstearat, Polysorbat 20, Laureth-23, PEG-40-Stearat, PEG-30-Glyceryllaurat, PEG-50-Stearat, PEG-100-Stearat, PEG-150-Laurat, Polyglyceryl-3-methylglucose Distearat, Ceteareth-12, Ceteareth-20 und Steareth-21, Ceteareth-6, PEG-40 Castor Oil, Natriumcetearylsulfat, Lecithin, Laureth-4-Phosphat, Propylenglykolstearat SE, PEG-25 Hydrogenated Castor Oil, PEG-54 Hydrogenated Castor Oil, Glycerylstearat SE, PEG-6 Caprylic/Capric Glycerides, Glyceryloleat und Propylenglykol, PEG-9-Stearat, Glyceryllanolat, Ceteth-2, Polysorbat 60, Glycerylmyristat, Glycerylisostearat und Polyglyceryl-3 Oleat, Glyceryllaurat, PEG-40-Sorbitanperoleat, Laureth-4, Glycerinmonostearat, Ceteareth-3, Lanolinsäure, Isostearylglycerylether, Cetearyl Alcohol und Natriumcetearylsulfat, Steareth-2, PEG-22-Dodecylglykolcopolymer, Polyglyceryl-2-PEG-4-Stearat, Pentaerythrithylisostearat, Polyglyceryl-3-Diisostearat, Sorbitanoleat und Hydrogenated Castor Oil und Cera alba und Stearinsäure, Natriumdihydroxycetylphosphat und Isopropylhydroxycetylether, Methylglucosesesquistearat, Steareth-2 und PEG-8-Distearat, Steareth-20, Isosteareth-20, Methylglucosedioleat, Sorbitanoleat und PEG-2 Hydrogenated Castor Oil und Ozokerit und Hydrogenated Castor Oil, PEG-2 Hydrogenated Castor Oil, PEG-45-/Dodecylglykolcopolymer, Methoxy PEG-22-/Dodecylglykolcopolymer, Hydrogenated Coco Glycerides, Polyglyceryl-4-Isostearat, PEG-40-Sorbitanperoleat, PEG-40-Sorbitanperisostearat, PEG-20-Glycerylstearat, PEG-8-Beeswax, Laurylmethiconcopolyol, Polyglyceryl-2-Laurat, Stearamidopropyl-PG-dimoniumchloridphosphat, PEG-7 Hydrogenated Castor Oil, Triethylcitrat, PEG-20 Methylglucosesesquistearat, Glycerylstearatcitrat, Cetylphosphat, Polyglycerolmethylglucosedistearat, Poloxamer 101, Kaliumcetylphosphat, Isosteareth-10, Oleth-20, Isoceteth-20, Glycerylisostearat, Polyglyceryl-3-Diisostearate, Cetearylalkohol und PEG-20-Stearat.

Der oder die Emulgatoren werden insbesondere vorzugsweise gewählt aus der Gruppe der Fettsäuren, welche ganz oder teilweise mit üblichen Alkalien (wie z. B. Natrium- und Kaliumhydroxid, Natrium- und Kaliumcarbonat sowie Mono- und Triethanolamin) neutralisiert sind. Besonders vorteilhaft sind beispielsweise Stearinsäure und Stearate, Isostearinsäure und Isostearate, Palmitinsäure und Palmitate sowie Myristinsäure und Myristate.

Die Emulgatoren werden erfindungsgemäß ferner vorzugsweise aus der Gruppe der gesättigten und/oder ungesättigten, verzweigten und/oder unverzweigten Fettalkohole mit 10 bis 40 Kohlenstoffatomen gewählt, besonders bevorzugt sind Butyloctanol, Butyldecanol, Hexyloctanol, Hexyldecanol, Octyldodecanol, Behenylalkohol ($C_{22}H_{45}OH$), Cetearylalkohol [eine Mischung aus Cetylalkohol ($C_{16}H_{33}OH$) und Stearylalkohol ($C_{18}H_{37}OH$)], Cetylarachidol [2-Hexadecyl-1-Eicosanol ($C_{36}H_{73}OH$),], Lanolinalkohole (Wollwachsalkohole, die die unverseifbare Alkoholfraktion des Wollwachses darstellen, die nach der Verseifung von Wollwachs erhalten wird) und/oder 2-Tetradecyloctadecanol ($C_{32}H_{65}OH$). Vorteilhafte Ausführungsformen der beiden letztgenannten Fettalkohole sind unter den Handelsnamen Isofol 36 und Isofol 32 bei Condea erhältlich.

Die Liste der genannten Emulgatoren, die im Sinne der vorliegenden Erfindung eingesetzt werden können, soll selbstverständlich nicht limitierend sein.

Die erfindungsgemäßen Zubereitungen können vorteilhaft auch ein oder mehrere Hydrokolloide enthalten.

Hydrokolloide sind Makromoleküle, die eine weitgehend lineare Gestalt haben und über intermolekulare Wechselwirkungskräfte verfügen, die Neben- und Hauptvalenzbindungen zwischen den einzelnen Molekülen und damit die Ausbildung eines netzartigen Gebildes ermöglichen. Sie sind teilweise wasserlösliche natürliche oder synthetische Polymere, die in wässrigen Systemen Gele oder viskose Lösungen bilden. Sie erhöhen die Viskosität des Wassers, indem sie entweder Wassermoleküle binden (Hydratation) oder aber das Wasser in ihre unter sich verflochtenen Makromoleküle aufnehmen und einhüllen, wobei sie gleichzeitig die Beweglichkeit des Wassers einschränken.

Die Gruppe der Hydrokolloide läßt sich wie folgt einteilen in:

- organische, natürliche Verbindungen, wie beispielsweise Agar-Agar, Carrageen, Tragant, Gummi arabicum, Alginate, Pektine, Polyosen, Guar-Mehl, Johannisbrotbaumkemmehl, Stärke, Dextrine, Gelatine, Casein,
- organische, abgewandelte Naturstoffe, wie z. B. Carboxymethylcellulose und andere Celluloseether, Hydroxyethyl- und -propylcellulose und dergleichen,
- organische, vollsynthetische Verbindungen, wie z. B. Polyacryl- und Polymethacryl-Verbindungen, Vinylpolymere, Polycarbonsäuren, Polyether, Polyimine, Polyamide,
- anorganische Verbindungen, wie z. B. Polykieselsäuren, Tonmineralien wie Montmorillonite, Zeolithe, Kieselsäuren.

Erfindungsgemäß bevorzugte Hydrokolloide sind beispielsweise Methylcellulosen, als welche die Methylether der Cellulose bezeichnet werden. Sie zeichnen sich durch die folgende Strukturformel aus

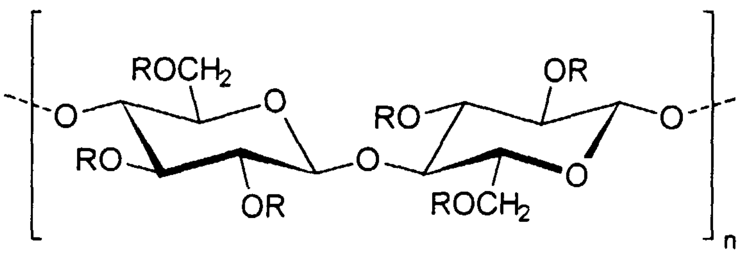

**Strukturformel II**

in der R ein Wasserstoff oder eine Methylgruppe darstellen kann.

Insbesondere vorteilhaft im Sinne der vorliegenden Erfindung sind die im allgemeinen ebenfalls als Methylcellulosen bezeichneten Cellulosemischether, die neben einem dominierenden Gehalt an Methyl- zusätzlich 2-Hydroxyethyl-, 2-Hydroxypropyl- oder 2-Hydroxybutyl-Gruppen enthalten. Besonders bevorzugt sind (Hydroxypropyl)methylcellulosen, beispielsweise die unter der Handeisbezeichnung Methocel E4M bei der Dow Chemical Comp. erhältlichen.

Erfindungsgemäß ferner vorteilhaft ist Natriumcarboxymethylcellulose, das Natrium-Salz des Glykolsäureethers der Cellulose, für welches R in Strukturformel II ein Wasserstoff und/oder $CH_2$-COONa darstellen kann. Besonders bevorzugt ist die unter der Handelsbezeichnung Natrosol Plus 330 CS bei Aqualon erhältliche, auch als Cellulose Gum bezeichnete Natriumcarboxymethylcellulose.

Bevorzugt im Sinne der vorliegenden Erfindung ist ferner Xanthan (CAS-Nr. 11138-66-2), auch Xanthan Gummi genannt, welches ein anionisches Heteropolysacchand ist, das in der Regel durch Fermentation aus Maiszukker gebildet und als Kaliumsalz isoliert wird. Es wird von Xanthomonas campestris und einigen anderen Species unter aeroben Bedingungen mit einem Molekulargewicht von $2\times10^6$ bis $24\times10^6$ produziert. Xanthan wird aus einer Kette mit β-1,4-gebundener Glucose (Cellulose) mit Seitenketten gebildet. Die Struktur der Untergruppen („repeated units") besteht aus Glucose, Mannose, Glucuronsäure, Acetat und Pyruvat.

Erfindungsgemäß vorteilhafte Hydrokolloide sind ferner Polymere der Acrylsäure, insbesondere solche, die aus der Gruppe der sogenannten Carbomere oder Carbopole (Carbopol® ist eigentlich eine eingetragene Marke der B. F. Goodrich Company) gewählt werden. Carbopole sind Verbindungen der allgemeinen Strukturformel

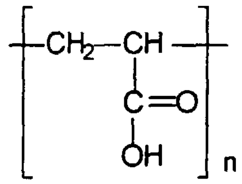

,

deren Molgewicht zwischen ca. 400 000 und mehr als 4 000 000 betragen kann. In die Gruppe der Carbopole gehören ferner Acrylat-Alkylacrylat-Copolymere, beispielsweise solche, die sich durch die folgende Struktur auszeichnen:

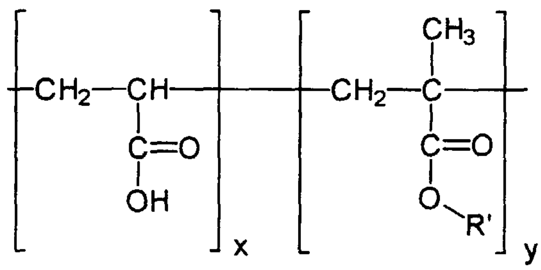

Darin stellen R' einen langkettigen Alkylrest und x und y Zahlen dar, welche den jeweiligen stöchiometrischen Anteil der jeweiligen Comonomere symbolisieren. Auch diese Carbopole sind vorteilhaft im Sinne der vorliegenden Erfindung.

Vorteilhafte Carbopole sind beispielsweise die Typen 907, 910, 934, 940, 941, 951, 954, 980, 981, 1342, 1382, 2984 und 5984, wobei diese Verbindungen einzeln oder in beliebigen Kombinationen untereinander vorliegen können. Besonders bevorzugt sind Carbopol 981, 1382 und 5984 (sowohl einzeln als auch in Kombination mit weiteren Hydrokolloiden).

Ferner vorteilhaft im Sinne der vorliegenden Erfindung sind die den Acrylat-Alkylacrylat-Copolymeren vergleichbaren Copolymere aus $C_{10-30}$-Alkylacrylaten und einem oder mehreren Monomeren der Acrylsäure, der Methacrylsäure oder deren Ester. Die INCI-Bezeichnung für solche Verbindungen ist „Acrylates/C 10-30 Alkyl Acrylate Crosspolymer". Insbesondere vorteilhaft sind die unter den Handelsbezeichnungen Pemulen TR1 und Pemulen TR2 bei der B. F. Goodrich Company erhältlichen.

Die Gesamtmenge an einem oder mehreren Hydrokolloiden wird in den fertigen kosmetischen oder dermatologischen Zubereitungen vorteilhaft kleiner als 1,0 Gew.-%, bevorzugt zwischen 0,01 und 0,5 Gew.-%, bezogen auf das Gesamtgewicht der Zubereitungen gewählt.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl,

z.B. Ethanol, Isopropanol, 1,2-Propandiol und Glycerin.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg - Ascorbylphosphat, Ascorbylacetat), Tocopherole und Derivate (z.B. Vitamin E - acetat), Vitamin A und Derivate (Vitamin A - palmitat) sowie Konyferyibenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Tnhydroxybutyrophenon, Hamsäure und deren Derivate, Mannose und deren Derivate, Zink und dessen Derivate (z.B. ZnO, $ZnSO_4$) Seien und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können öllösliche Antioxidantien eingesetzt werden.

Eine erstaunliche Eigenschaft der vorliegenden Erfindung ist, daß erfindungsgemäße Zubereitungen sehr gute Vehikel für kosmetische oder dermatologische Wirkstoffe in die Haut sind, wobei bevorzugte Wirkstoffe Antioxidantien sind, welche die Haut vor oxidativer Beanspruchung schützen können. Bevorzugte Antioxidantien sind dabei Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist dem Fachmann natürlich bekannt, daß kosmetische Zubereitungen zumeist nicht ohne die üblichen Hilfs- und Zusatzstoffe denkbar sind. Die erfindungsgemäßen kosmetischen und dermatologischen Zubereitungen können dementsprechend ferner kosmetische Hilfsstoffe enthalten, wie sie üblicherweise in solchen Zubereitungen verwendet werden, beispielsweise Konsistenzgeber, Füllstoffe, Konservierungsmittel, Parfüme, Substanzen zum Verhindern des Schäumens, Farbstoffe, weitere oberflächenaktive Substanzen, weichmachende, anfeuchtende und/oder feuchthaltende Substanzen, entzündungshemmende Substanzen, zusätzliche Wirkstoffe wie Vitamine oder Proteine, Lichtschutzmittel, Insektenrepellentien, Bakterizide, Viruzide, Wasser, Salze, antimikrobiell, proteolytisch oder keratolytisch wirksame Substanzen, Medikamente oder andere übliche Bestandteile einer kosmetischen oder dermatologischen Formulierung wie Alkohole, Polyole, Polymere, Schaumstabilisatoren, organische Lösungsmittel oder auch Elektrolyte.

Mutatis mutandis gelten entsprechende Anforderungen an die Formulierung medizinischer Zubereitungen.

Die erfindungsgemäßen O/W-Emulsionen können als Grundlage für kosmetische oder dermatologische Formulierungen dienen. Diese können wie üblich zusammengesetzt sein und beispielsweise zur Behandlung und der Pflege der Haut und/oder der Haare, als Lippenpflegeprodukt, als Deoprodukt und als Schmink- bzw. Abschminkprodukt in der dekorativen Kosmetik oder als Lichtschutzpräparat dienen. Zur Anwendung werden die erfindungsgemäßen kosmetischen und dermatologischen Zubereitungen in der für Kosmetika oder Dermatika üblichen Weise auf die Haut und/oder die Haare in ausreichender Menge aufgebracht..

Entsprechend können kosmetische oder topische dermatologische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Die dünnflüssigen kosmetischen oder dermatologischen Mittel gemäß der Erfindung können beispielsweise als aus Aerosolbehältern, Quetschflaschen oder durch eine Pumpvorrichtung versprühbare Präparate vorliegen oder in Form einer mittels Roll-on-Vorrichtungen auftragbaren flüssigen Zusammensetzung, jedoch auch in Form einer aus normalen Flaschen und Behältern auftragbaren Emulsion.

Als Treibmittel für aus Aerosolbehältern versprühbare kosmetische oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Günstig sind auch solche kosmetischen und dermatologischen Zubereitungen, die in der Form eines Sonnenschutzmittels vorliegen. Vorzugsweise enthalten diese zusätzlich mindestens eine UV-A-Filtersubstanz und/oder mindestens eine UV-B-Filtersubstanz.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden z.B. in Tagescrèmes gewöhnlich UV-A- bzw. UV-B-Filtersubstanzen eingearbeitet.

Auch stellen UV-Schutzsubstanzen, ebenso wie Antioxidantien und, gewünschtenfalls, Konservierungsstoffe, einen wirksamen Schutz der Zubereitungen selbst gegen Verderb dar.

Vorteilhaft enthalten erfindungsgemäße Zubereitungen Substanzen, die UV-Strahlung im UV-A- und/oder UV-B-Bereich absorbieren, wobei die Gesamtmenge der Filtersubstanzen z. B. 0,1 Gew.-% bis 30 Gew.-%, vorzugsweise 0,5 bis 20 Gew.-%, insbesondere 1,0 bis 15,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Weitere vorteilhafte UV-A-Filtersubstanzen sind die Phenylen-1,4-bis-(2-benzimidazyl)-3,3'-5,5'-tetrasulfonsäure:

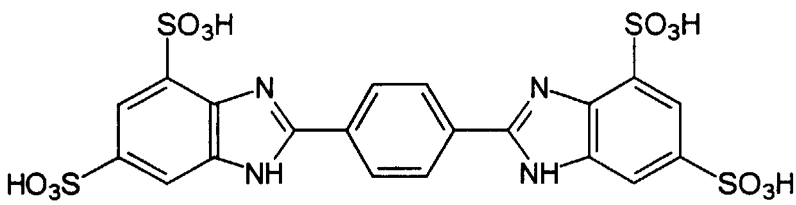

und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz:

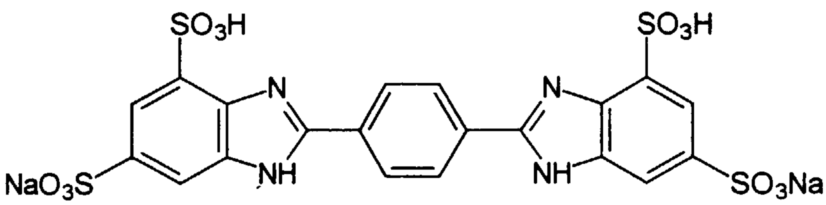

sowie das 1,4-di(2-oxo-10-Sulfo-3-bornylidenmethyl)-Benzol und dessen Salze (besonders die entsprechenden 10-Sulfato-verbindungen, insbesondere das entsprechende Natrium-, Kalium- oder Triethanolammonium-Salz), das auch

als Benzol-1,4-di(2-oxo-3-bornylidenmethyl-10-sulfonsäure) bezeichnet wird und sich durch die folgende Struktur auszeichnet:

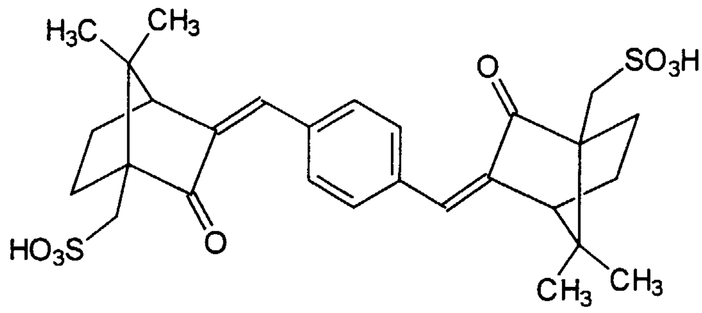

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Bis-Resorcinyltriazinderivate mit der folgenden Struktur:

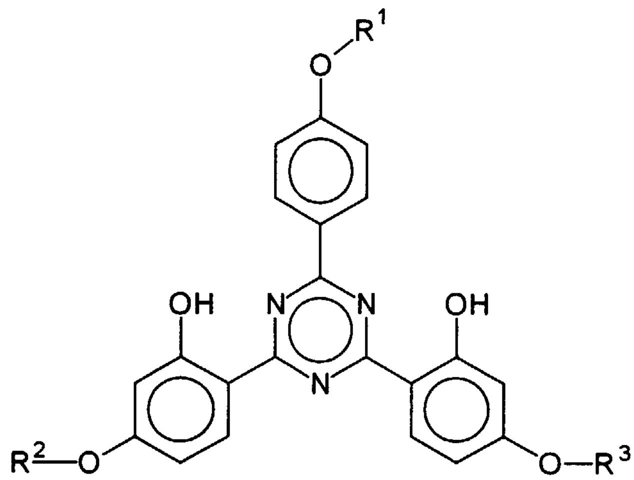

wobei $R^1$, $R^2$ und $R^3$ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist, und das 4,4',4"-(1,3,5-Triazin-2,4,6-triyltriimino)-tris-benzoësäure-tris(2-ethylhexylester), synonym: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird.

Auch andere UV-Filtersubstanzen, welche das Strukturmotiv

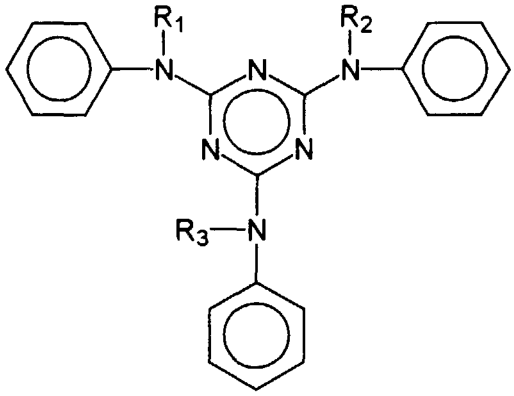

aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel

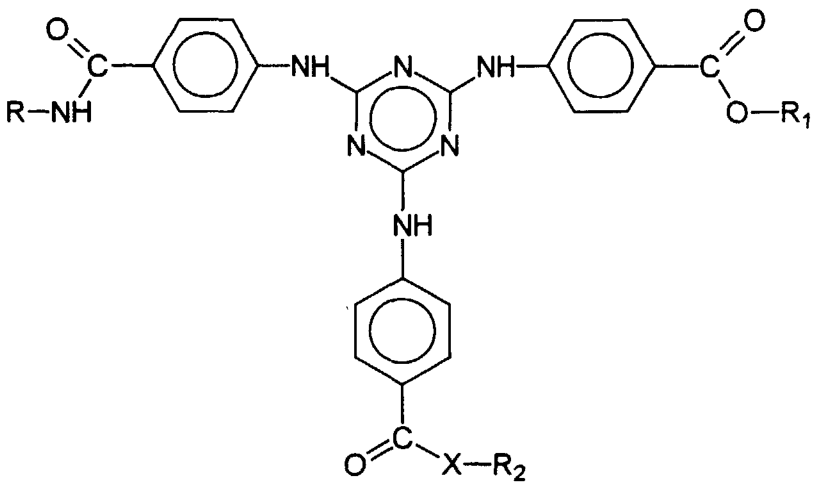

wiedergegeben wird, wobei

R einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt,

X ein Sauerstoffatom oder eine NH-Gruppe darstellt,

$R_1$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

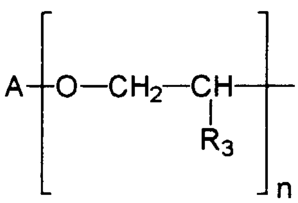

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,

$R_3$ ein Wasserstoffatom oder eine Methylgruppe darstellt,

n eine Zahl von 1 bis 10 darstellt,

$R_2$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt, wenn X die NH-Gruppe darstellt, und einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

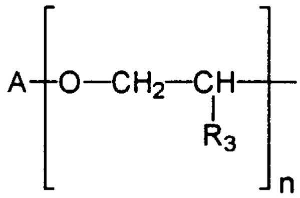

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,

$R_3$ ein Wasserstoffatom oder eine Methylgruppe darstellt,

n eine Zahl von 1 bis 10 darstellt,

wenn X ein Sauerstoffatom darstellt.

Besonders bevorzugte UV-Filtersubstanz im Sinne der vorliegenden Erfindung ist ferner ein unsymmetrisch substituiertes s-Triazin, dessen chemische Struktur durch die Formel

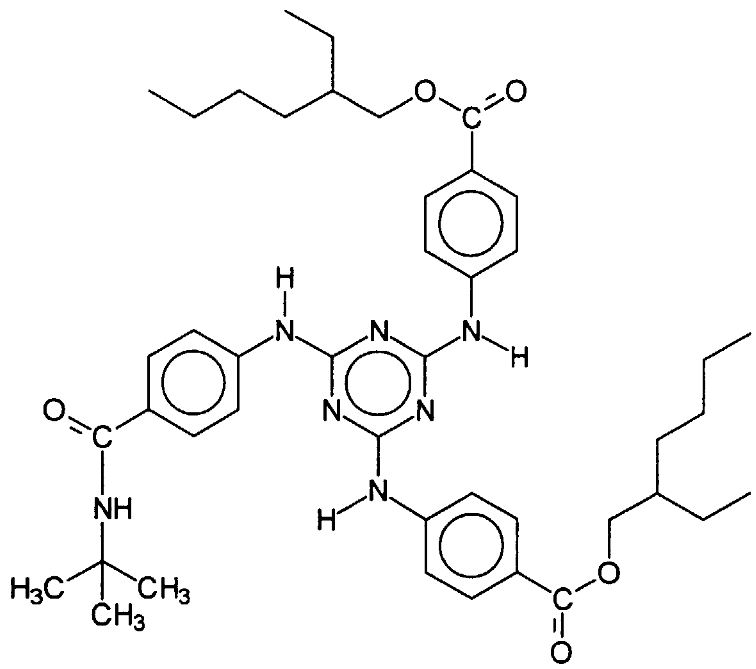

wiedergegeben wird und welche im Folgenden auch als Dioctylbutylamidotriazon bezeichnet wird.

Auch in der Europäischen Offenlegungsschrift 775 698 werden bevorzugt einzusetzende Bis-Resorcinyltriazinderivate beschrieben, deren chemische Struktur durch die generische Formel

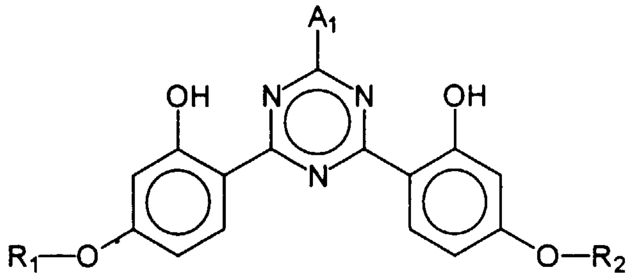

wiedergegeben wird, wobei $R_1$, $R_2$ und $A_1$ verschiedenste organische Reste repräsentieren.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{(4-(3-(2-propyloxy)-2-hydroxy-propyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin.

Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol), welches durch die chemische Strukturformel

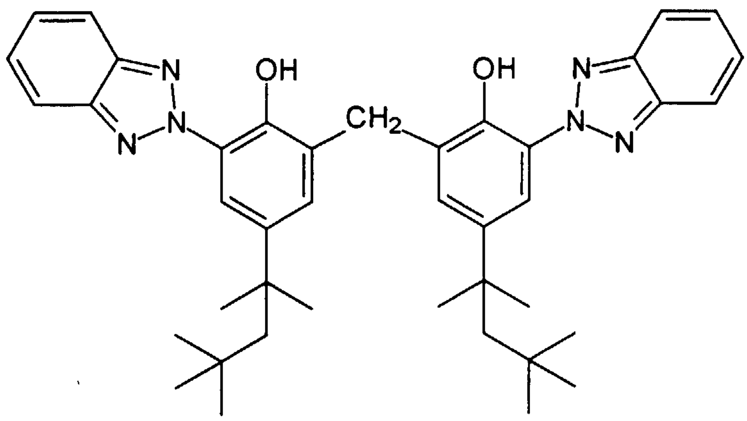

gekennzeichnet ist und unter der Handeisbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane, welches durch die chemische Strukturformel

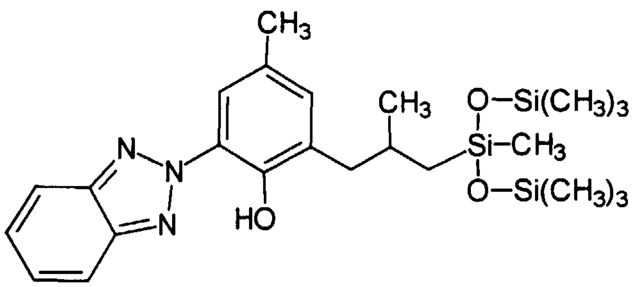

gekennzeichnet ist.

Die UV-B-Filter können öllöslich oder wasserlöslich sein. Vorteilhafte öllösliche UV-B-Filtersubstanzen sind

z. B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
- sowie an Polymere gebundene UV-Filter.

Vorteilhafte wasserlösliche UV-B-Filtersubstanzen sind z. B.:

- Salze der 2-Phenylbenzimidazol-5-sulfonsäure, wie ihr Natrium-, Kalium- oder ihr Triethanolammonium-Salz, sowie die Sulfonsäure selbst;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist und sich durch folgende Struktur auszeichnet:

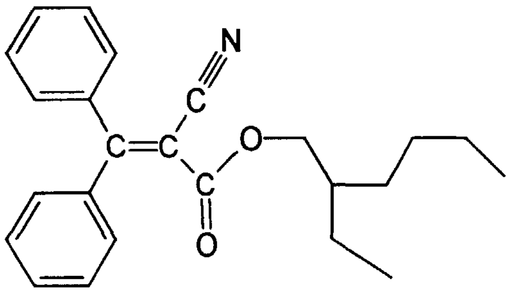

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Ferner kann es gegebenenfalls von Vorteil sein, erfindungsgemäß weitere UV-A- und/oder UV-B-Filter in kosmetische oder dermatologische Zubereitungen einzuarbeiten, beispielsweise bestimmte Salicylsäurederivate wie 4-Isopropylbenzylsalicylat, 2-Ethylhexylsalicylat (= Octylsalicylat), Homomenthylsalicylat.

Die Liste der genannten UV-Filter, die im Sinne der vorliegenden Erfindung eingesetzt werden können, soll selbstverständlich nicht limitierend sein.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

**Beispiele:**

| | 1 | 2 | 3 | 4 | 5 |
|---|---|---|---|---|---|
| Stearinsäure | 0,3 | | | | |

(fortgesetzt)

| | 1 | 2 | 3 | 4 | 5 |
|---|---|---|---|---|---|
| Cetyldimethiconcopolyol | | | | 0,1 | |
| Glycerylstearatcitrat | | | | 0,5 | 0,3 |
| Dimethicon | 2 | 2,5 | | 2 | |
| Phenyltrimethicon | 2 | | 3 | | |
| Caprylic/Caprictriglycerid | 5 | 5 | | 5 | 5 |
| $C_{12-15}$ Alkylbenzoat | | 5 | 5 | 5 | |
| Dicaprylylether | 5 | 5 | 5 | | 5 |
| Butylenglykoldicaprylat/Caprat | | | 5 | | 2 |
| Mineralöl | 4 | | | | |
| Cetylpalmitat | | | | | 0,5 |
| Vitamin E-Acetat | 0,5 | | 0,5 | | 0,5 |
| Dioctylbutamidotriazon | | | 1 | | |
| Anisotriazin | | | | 2 | |
| Octylmethoxycinnamat | | | | 8 | |
| Octyltriazon | | | 1 | | |
| Methylbenzylidencampher | | | 2 | | |
| Butylmethoxydibenzoylmethan | | | 1 | | |
| Eusolex T2000 ® | 2,8 | 2,0 | 2,0 | | |
| Aerosil 380 ® | | 0,6 | 0,6 | 0,4 | |
| Zinkoxid | | | | 2,5 | |
| Distärkephosphat | | | | | 8,5 |
| Konservierung | 0,5 | 0,5 | 0,5 | 0,5 | 0,5 |
| | | | | | |
| Glycerin | 3 | 6 | 10 | 6 | 10 |
| Xanthan Gummi | | | | 0,1 | 0,1 |
| Pemulen TR1 ® | | | 0,1 | | |
| Phenylbenzimidazolsulfonsäure | | | 2 | | |
| Natronlauge 45% | | | 1,2 | | |
| Wasser | ad 100 | ad 100 | ad 100 | ad 100 | ad 100 |

**Patentansprüche**

1. Dünnflüssige kosmetische oder dermatologische Zubereitungen vom Typ Öl-in-Wasser, welche eine Ölphase, in welche hydrophobe und/oder amphiphile Feststoffe eingearbeitet sind, und eine Wasserphase enthalten, wobei die Dichtedifferenz zwischen der Ölphase und der Wasserphase (bestimmbar mit einem rechnenden digitalen Dichtemesser vom Typ DMA 45 der Firma chempro/PA bei 25 °C) nicht größer als 0,01 g • $cm^{-3}$ ist.

2. Zubereitung nach Anspruch 1, dadurch gekennzeichnet, daß zusätzlich weitere kosmetische oder pharmazeutische Hufs-, Zusatz- und/oder Wirkstoffe enthalten sind.

3. Zubereitung nach einem der vorhergehenden Ansprüche, dadurch gekennzeichnet, daß die Viskosität dieser Zubereitung kleiner als 2.000 mPa • s, insbesondere kleiner als 1.500 mPa • s (bestimmbar mit einem Haake Viskotester VT-02 bei 25 °C) ist.

**4.** Zubereitung nach einem der vorhergehenden Ansprüche, dadurch gekennzeichnet, daß die Zubereitung sprühbar ist.

**5.** Zubereitung nach einem der vorhergehenden Ansprüche, dadurch gekennzeichnet, daß der Durchmesser der Öltröpfchen im Durchschnitt kleiner als 50 µm ist.

**6.** Zubereitung nach einem der vorhergehenden Ansprüche, dadurch gekennzeichnet, daß die Dichte der Gesamtformulierung größer als 0,9 $g \cdot cm^{-3}$, insbesondere größer als 0,95 $g \cdot cm^{-3}$ ist.

**7.** Zubereitung nach einem der vorhergehenden Ansprüche, dadurch gekennzeichnet, daß der Gehalt an einem oder mehreren Emulgatoren kleiner als 1 Gew.-%, insbesondere kleiner als 0,5 Gew.-% ist, jeweils bezogen auf das Gesamtgewicht der Zubereitung.

**8.** Zubereitung nach einem der vorhergehenden Ansprüche, dadurch gekennzeichnet, daß sie emulgatorfrei ist.

**9.** Zubereitung nach einem der vorhergehenden Ansprüche, dadurch gekennzeichnet, daß der Gehalt an einem oder mehreren Verdickungsmitteln zwischen 0,05 Gew.-% und 0,15 Gew.-% ist, bezogen auf das Gesamtgewicht der Formulierung.

**10.** Verwendung von Zubereitungen nach einem der vorhergehenden Ansprüche als kosmetisches oder dermatologisches Lichtschutzmittel.

**11.** Verfahren zur Stabilisierung von O/W-Formulierungen, dadurch gekennzeichnet, daß die Dichte der Ölphase durch Zugabe von hydrophoben und/oder amphiphilen Feststoffen der Dichte der Wasserphase derart angeglichen wird, daß die Dichtedifferenz der beiden Phasen (bestimmbar mit einem rechnenden digitalen Dichtemesser vom Typ DMA 45 der Firma chempro/PA bei 25 °C) nicht größer als 0,01 $g \cdot cm^{-3}$ ist.